# NEUE EUROPÄISCHE PATENTSCHRIFT

(11) **EP 0 197 987 B2**
(45) Veröffentlichungstag und Bekanntmachung des Hinweises auf die Entscheidung über den Einspruch: **29.06.1994**
(45) Hinweis auf die Patenterteilung: 20.09.1989
(21) Anmeldenummer: 85904955.3
(22) Anmeldetag: 18.10.1985
(51) Int. Cl.: A61K 7/00, A61K 9/10

(54) **VERFAHREN ZUR HERSTELLUNG VON LÖSUNGEN VON UMGEKEHRTEN MIZELLEN**
PROCESS FOR PREPARING A SOLUTION OF INVERTED MICELLAE
PROCEDE POUR LA PREPARATION D'UNE SOLUTION DE MICELLES INVERSEES

(30) Priorität: 18.10.1984 CH 4983/84
(43) Veröffentlichungstag der Anmeldung: 22.10.1986
(73) Patentinhaber: ZAMBON S.p.A., I-20091 Bresso (Milano) (IT)
(72) Erfinder: MENEGATTI, Enea, I-44100 Ferrara (IT); MENDLER, Markus, ETH Zentrum CH-8092 Zürich (CH); PANDE, Ajay, ETH Zentrum CH-8092 Zürich (CH); JÄCKLE, Hans, ETH Zentrum CH-8092 Zürich (CH); LUISI, Pier Luigi, CH-8092 Zürich (CH)
(74) Vertreter: Monsch, René
(86) Internationale Anmeldenummer: CH8500154
(87) Internationale Veröffentlichungsnummer: WO8602264

(56) Entgegenhaltungen:
- EP-A- 0 102 324
- EP-A- 0 129 435
- EP-A- 0 130 577
- EP-A- 0 140 085
- EP-A- 0 171 084
- DE-A- 2 928 040
- DE-A- 3 212 053
- FR-M- 4 690
- GB-A- 2 046 096
- US-A- 3 492 399
- Journal of Food Science, Vol. 36 (1971), 248-250 "Solubilisation of aqueous solutions in nonpolar liquids"
- Cosmetics & Toileteries, Vol. 97 (Juni 1982), 55-58 "Microemulsion"
- J. Soc. Cosmet. Chem. 34, 335-350 (Nov. 1983) "Microemulsions: Evolving technology for cosmetic applications".
- Top. Pharm. Sci. Int. Congr. Pharm. Sci. F.I.P., 43rd, 243-255 Edited by: Breimer, Douwe D.; Speiser, Peter. Elsevier, Amsterdam (NL) 1983; Microemulsions: Proteins and nucleic acids as guest molecules.
- Chemical Abstracts, Vol. 102 (1985), 31938r
- Chemical Abstracts, Vol. 99 (1983), 128152n
- "Water-in-oil microemulsions and microemulsiongels: Physical characterization and chemical properties", G.J. Haering, Diss. ETH No 8950, Zürich-CH, 1989

## Beschreibung

Die vorliegende Erfindung betrifft ein Verfahren zur Herstellung von Lösungen von umgekehrten Mizellen, deren Teilchendurchmesser der wässrigen Phase 200 A nicht übersteigt, die dann auch Mikroemulsionen genannt werden, wenn der Gehalt an Wasser im System relativ gross ist, in Form von Gelen. Das erfindungsgemässe Verfahren ist dadurch gekennzeichnet, dass man in eine Mischung aus wenigstens einem biokompatiblen natürlichen organischen Lösungsmittel und wenigstens einem Tensid, sowie gegebenenfalls einem Cotensid oder einem Coadjuvans, und Wasser, sowie einem in Wasser gelösten Stoff, der eine therapeutische oder kosmetische Aktivität aufweist, ein gelbildendes Mittel einverleibt, vorzugsweise ein gelbildendes Mittel auf Basis eines Polysaccharides oder Gelatine.

In den nach dem erfindungsgemässen Verfahren hergestellten Lösungen von umgekehrten Mizellen in Gelform enthält das biokompatible natürliche organische Lösungsmittel, beispielsweise ein Kohlenwasserstoff, die umgekehrten Mizellen der wässrigen Phase, deren Teitchendurchmesser 200 A nicht übersteigt. In dieser wässrigen Mikrophase sind hydrophile Substanzen, die eine therapeutische oder kosmetische Aktivität aufweisen, gelöst.

In den erhaltenen Gelen ist der in der wässrigen Phase gelöste Stoff mit therapeutischer oder kosmetischer Aktivität gleichmässig in einer öligen Phase, nämlich dem biokompatiblen organischen Lösungemittel solubilisiert, wobei als Beispiel für derartige ölige Lösungsmittel Isopropylpalmitat, Mygliol oder natürliche ölige Substanzen, oder essentielle Oele, oder natürliche Terpene oder Kohlenwasserstoffe wie Squalan, Squalen und Derivate genannt seien

Es ist bekannt, dass einige Tenside stabile spherische Aggregate in apolaren Lösungen bilden können. Die polaren Köpfe des Tensids weisen in diesen Aggregaten nach innen; es entsteht somit ein polarer Kern, der seinerseits Wasser solubilisieren kann. Im Innern dieser wässrigen Mikrophase, deren Grösse zwischen 10-200 A liegt, können hydrophile Stoffe solubilisiert werden. Es bilden sich auf diese Weise thermodynamisch stabile und klare Lösungen. Die chemischen und physikalischen Eigenschaften solcher Aggregate wurden von Autoren wie Fendler, Menger, Eicke in den letzten 10 Jahren ausführlich untersucht; siehe J.H. Fendler, "Membrane mimetic chemistry", John Wiley and Sons, N.Y. 1982; F.M. Menger et al., J. Amer. Chem. Soc. 95, 286 (1983); H.F. Heicke und J. Rehak, Helv. Chim. Acta 59, 2883 (1976); H.F. Heicke Topics Curr. Chem., 87, 85 (1980). Für eine detaillierte Beschreibung empfehlen wir das Buch "Reverse Micelles" Plenum Press 1984, New York, herausgegeben von P.L. Luisi und B. Straub. Diese Wasser-in-Oel-Mikroemulsionen, in welchen die Hauptkomponente ein organisches Lösungsmittel ist, unterscheiden sich wesentlich von den bekann- teren Oel-in-Wasser-Mikroemulsionen, in welchen die Hauptkomponente Wasser ist.

Betreffend die grundsätzlichen Unterschiede zwischen "Wasser-in-Oel"- und "Oel-in-Wasser"-Mikroemulsionen sei verwiesen auf
- J.H. Fendler und E.J. Fendler, "Catalysis in micellar and macromolecular systems", Academic Press, New York, 1975;
- "Solution, BehaviorofSurfactants", K.L. Mittal und E.J. Fendler, Volume 1, Plenum Press, New York, 1982; und
- "Surfactants in Solution", K.L. Mittal und B. Lindman, Vol. 1 bis 3, Plenum Press, New York, 1984.

Für diese Oel-in-Wasser-Mikroemulsionen sind schon einige pharmazeutische Anwendungen zitiert worden; siehe z.B. die FR-A-4960 (med).

Es gibt dagegen wenige pharmazeutische Anwen- dungen mit Ausnahme von der Anwendung für die Vorbereitung von "Nanokapseln", wie von Speiser in Zürich vorgeschlagen; siehe: P.P. Speiser, in "Applied Biology and Therapeutics" 6, Lingle und Breimer editors, Elsevier, N.Y. 1983;
- J. Ziegenmayer und C. Führer, Acta Pharmaceutica Technologica 26(4) 1980, Seiten 273 bis 275; und
- M.C. Martini, M.F. Bobin, H. Flandin, F. Caillaud und J. Cotte, J. Pharm. Belg. 1984, 39, 6, Seiten 348 bis 354.

Nachfolgend werden noch einige zum Stand der Technik gehörende Dokumente abgehandelt.

In der Patentschrift US-A-3 492 399 wird eine Wasser-in-Oel-Emulsion beschrieben; d.h. eine durch die Einwirkung von mechanischen Kräften erhaltene Mischung von Oel und Wasser in etwa den gleichen Mengenverhältnissen (also keine Mikroemulsion). Dabei liegt die Teilchengrösse der wässrigen Phase zwischen etwa 0,1 bis 10 Mikrometer. Das heisst, diese Teilchen sind 100 bis 1000 mal grösser als jene der erfindungsgemäss erhältlichen Wasser-in-Oel-Mikroemulsionen die in Form von Gelen vorliegen. Zudem ist ein Emulgator zwingend in den in dieser US-Patentschrift beschriebenen Emulsionen vorhanden. Es werden 1 bis 2 Volumenteile Wasser pro Teil Oel-Phase eingesetzt.

In der Patentschrift FR-A-4 960 (med.) wird ebenfalls eine Wasser-in-Oel-Emulsion beschrieben. Gemäss den Beispielen 1 und 6 dieser Schrift liegt die Teilchengrösse zwischen 25 bis 30 Mikrometer, respektiv bei 50 Mikrometer. Sie ist somit noch grösserals die in der US-A-3492 399 erwähnte Teilchengrösse. Ein Emulgator ist ebenfalls immer zwingend vorhanden. Diese Emulsion wird durch Anwendung von mechanischen Kräften erhalten (siehe Seite 2, linke Spalte, Ende des zweitletzten Abschnittes), wogegen sich die erfindungsgemässen umgekehrten Mizellen spontan ohne Einwirkung von mechanischen Kräften bilden.

Die Europäische Patentanmeldung mit der Publikationsnummer EP-A-171 084 handelt von einer Oel-in-Wasser-Mikroemulsion, worin die Hauptkomponente Wasser ist. Bei den erfindungsgemässen umgekehrten Mizellen ist die Hauptkomponente ein organisches Lösungsmittel ("Oel").

Betreffend die grundsätzlichen Unterschiede zwischen "Wasser-in-Oel"- und "Oel-in-Wasser"-Mikroemulsionen sei auf die obigen Ausführungen verwiesen.

Die Europäische Patentanmeldung mit der Publikationsnummer EP-A-129 435 handelt wiederum von einer Emulsion, die einen definierten Ester von Flurbioprofen enthält. Diese Emulsionen sind vom Oel-in-Wasser-Typus; siehe Seite 4, Zeile 16, Seite 5, Zeilen 21 und 22, sowie Seite 15, mittlerer Abschnitt.

Gemäss einer Ausführungsart des erfindungsgemässen Verfahrens zur Herstellung von Lösungen von umgekehrten Mizellen, deren Teilchendurchmesser der wässrigen Phase 200 A nicht übersteigt, die dann auch Mikroemulsionen genannt werden, wenn der Gehalt an Wasser im Syste, relativ gross ist, in Form von Gelen, wird zunächst eine Mischung aus wenigstens einem biokompatiblen natürlichen organischen Lösungsmittel und wenigstens einem Tensid, sowie gegebenenfalls einem Cotensid oder einem Coadiuvanten, hergestellt, und anschliessend wird ein in Wasser gelöster Stoff, der eine therapeutische oder kosmetische Aktivität aufweist, hinzugegeben, wobei man Lösungen von umgekehrten Mizellen erhält und dann wird Gelatine oder ein Polysaccharid zugefügt und man lässt dieses Gemisch gelieren.

Das wichtigste Kennzeichen des erfindungsgemäss hergestellten Systems besteht darin, dass die hydrophilen Moleküle, inbegriffen Enzyme, Nukleinsäure und Polysaccharide, in organischen Lösungsmitteln, beispielsweise Kohlenwasserstoffen solubilisiert werden können. Die in Kohlenwasserstoff gelösten Enzyme können mit Hilfe der umgekehrten Mizellen ihre Aktivität nicht verlieren; siehe P.L. Luisi und R. Wolf, "Solution behaviour of surfactans" vol. 12, Mittel und Fendler eds., Plenum Press 1982 und R. Wolf und P.L. Luisi, Bioch. Bioph. Res. Comm. 89, 209 (1979).

Das am meisten untersuchte System mit biologischen Substanzen ist aus Isooctan/AOT/Wasser gebildet. AOT ist die Abkürzung für das wohlbekannte biokompata- bile Tensid, Natrium-1,2-bis (2-Ethylhe- xyloxycarbonyl)-1-ethansulfonat.

Das organische Lösungsmittel, das bis jetzt zur Solubilisierung von Proteinen oder Peptiden in mizellaren umgekehrten Lösungen verwendet wurde, ist mit wenigen Ausnahmen ein Kohlenwasserstoff von niedrigem Molekulargewicht und, als solcher, nicht ideal für eine direkte pharmazeutische Anwendung.

In diesem Verfahren wird beschrieben, wie Gele von Arzneimitteln oder Verbindungen, die eine kosmetische Aktivitätaufweisen können, mittels biokompatiblen Lösungsmitteln wie Squalan, Miglyol®, Estern von palmitinsäure (insbesondere Isopropylpalmitat), Pflanzenöle, Vaselinöl, hergestellt werden. Das Tensid AOT wird für mehrere in dieser Beschreibung angegebene Beispiele verwendet. Das Verfahren ist auch mit anderen Tensiden durchfuhrbar, wie in den Beispielen und in den Ansprüchen dieser Unterlagen gezeigt wird.

Lösungen von umgekehrten Mizellen sind bereits bekannt, die erfindungsgemäss erhältlichen mizellaren Lösungen in Form von Gelen, die durch Einverleibung eines gelbildenden Mittels erhalten werden, sind aber bis jetzt in der Literatur noch nicht beschrieben worden. Diese Gele sind thermodynamisch stabil und sie enthalten in der wässrigen Phase, deren Teilchendurchmesser200 A nicht übersteigt, einen in dieser wässrigen Phase gelösten Stoff, der eine therapeutische oder kosmetische Wirksamkeit besitzt.

Die erfindungsgemäss erhältlichen Gele sind in vielen Bereichen vorteilhaft, da sie die Aufnahme des Wirkstoffes mit therapeutischer oder kosmetischer Aktivität erleichtern, und zwar insbesondere
i) die gastrointestinale Absorption von Arzneimitteln
ii) die transdermale Absorption
iii) die kosmetische Anwendung.

Die Arzneimittel- oder Kosmetikakonzentrationen in den gelformigen mizellaren Lösungen können spektrophotometrisch bestimmt werden (UV oder ORD). Es können auch chromatographische (z.B. HPLC) oder elektrophoretische Methoden angewendet werden.

Im Falle der Enzyme kann die Konzentration auf eine einfache Weise aus ihrer Aktivität berechnetwerden, die auf ähnliche Weise für wässrige Lösungen bestimmt wird; siehe P.L. Luisi und R. Wolf, "Solution behaviour of surfactans" vol. 12, Mittel and Fendler eds., Plenum Press 1982.

Sollte es notwendig sein, die pharmakologische Aktivität abzuschätzen (z.B. im Falle von Hormonen), kann das Arzneimittel einfach von der mizellaren in eine wässrige Lösung mittels "Backward Transfer" Methode versetzt werden; siehe P.L. Luisi et al. in "Topics in Pharmaceutical Science", Breimer and Speiser eds. Elsevier (1983).

Das Urogastron (human Epidermal Growth Factor, hEGF) ist ein Hormon mit neuer Epithel-bildender Aktivität, das die saure Magenabsonderung hemmen kann; siehe R. Hori et al. Chem. Pharm. Bull. 25,1974 (1977); R. Hori et al. Japan Kokai, 76, 95 (1975). Dank dieser Eigenschaften stellt das hEGF ein gutes Mittel gegen Geschwüre dar. Man hat festgestellt, dass mit der Zugabe von Olivenöl oder einem Glycerol-Derivat die gastrointestinale Absorption zunimmt. Andererseits ist es bekannt, dass einige Tenside die Geschwindigkeit der Absorption beschleunigen können; siehe G. Levy et al. J. Pharm. Sci., 55, 394 (1966). Die in Form von Gelen vorliegenden umgekehrten Mizellen (oft auch Mikroemulsionen Wasser/Oel benannt) stellen den idealen Träger für diese Hormone dar.

Das Urogastron ist in in den Mikroemulsionsgelen einfacherweise in derwässrigen Mikrophase eingegliedert und kann aus dem organischen Lösungsmittel diffundieren.

Der bedeutendste Vorteil dieses Systems besteht in der Tatsache, dass der teuere biologische Stoff, in unserem Fall Urogastron, nicht verloren geht, weil das Hormon nur im Wasser im Innern der invertierten Mizellen enthalten ist.

Die Herstellung der mizellaren Lösungen, die Urogastron oder seine Bruchteile, oder das durch kovalente Bindung mit Methoxypolyethylenglykol (PEG) modifizierte Hormon enthalten, verfolgt das klassische Verfahren zur Vorbereitung mizellarer Lösungen; siehe: P.L. Luisi und R. Wolf, "Solution behaviour of surfactans" vol. 12, Mittel and Fendler eds., Plenum Press 1982. Das Tensid (z.B. AOT) wird im Lösungsmittel gelöst, so dass eine Tensidlösung zwischen 50 mM und 300 mM entsteht. Die Auflösung geschieht bei Raumtemperatur und ohne Sonifizierung. Zu dieser organischen Lösung kann man die wässrige Lösung des Biopolymers zugeben; z.B. 1 ml einer gepufferten Lösung von Urogastron (Phosphat 50 mM pH 7.0) werden zu 100 ml der vorherigen organischen AOT-Lösung zugefügt. Der Anteil von Wasser im System kann bis zu 30% betragen, insbesondere zwischen 0,03% bis 3% (v:v) variieren.

Unter diesen Bedingungen entsteht beim Schütteln von Hand während einigen Minuten und ohne Sonifizierung eine klare und durchsichtige Lösung. Als organische Lösungsmittel kommen - wie schon erwähnt - niedermolekulare Kohlenwasserstoffe, fluorierte Kohlenwasserstoffe, natürliche und pflanzliche Oele, Squalan, Squalen, Ester von Palmitinsäure und von anderen Fettsäuren, Miglyol® in Frage. Die schlussendliche Konzentration des Biopolymers (in unserem Fall Urogastron) kann innerhalb der endgültigen Lösung in einem breiten Bereich variieren, gemäss der wässrigen Startlösung und dem Gebrauch. Normalerweise variiert sie zwischen 0,1 - 10 mg/ml, insbesondere 0,1 - 1,0 mg/ml (Konzentration bezüglich totalem Volumen). Der pH-Wert kann auch breit variiert werden, aber normalerweise werden Lösungen mit einem pH-Wert zwischen 5 und 7 verwendet.

Die auf diese Weise vorbereiteten Lösungen sind für lange Zeit stabil, im besonderen, wenn sie im Kühlschrank (0-2° C) aufbewahrt werden. Für eine verlängerte Aufbewahrung werden die Lösungen unter 0° C abgekühlt. Ein Niederschlag wird nur bei höherer Wasserkonzentration (zwischen 5% - 10%) beobachtet, aber das System ist reversibel.

In diesen Lösungen von umgekehrten Mizellen gefriert das Wasser bei niedrigen Wasseranteilen (unter 3%), nicht bei 0° C.

Durch Einverleibung des gelbildenden Mittels in diese Lösungen von umgekehrten Mizellen, beziehungsweise Mikroemulsionen, sind erfindungsgemäss die Mikroemulsionsgele erhältlich.

Medroxyprogesteronacetat wird heutzutage in der Therapie einiger hormonabhängiger Krebse durch intramuskuläre Spritzen verwendet. Das häufige Auftreten von Geschwüren stellt, verbunden mit dieser Art von Verabreichung, Probleme dar. Eine orale Verabreichung wäre bequemer, wenn die Probleme mit einer kleinen Absorption gelöst werden könnten. Die Verwendung von umgekehrten Mizellen, in Form von Gelen hergestellt mittels Verwendung eines Lösungsmittels als kontinuierliche Phase, in der das Medroxyprogesteronacetat schwer löslich ist, beschleunigt die Absorptions-Geschwindigkeit, möglicherweise wegen direkter Einwirkung der Mizellen auf die biologische Membrane, in der die Permeabilität zum Arzneimittel erhöht wird.

Die Vorbereitung der mizellaren Lösungen, enthaltend Medroxyprogesteronacetat, verfolgt das klassische Verfahren der Zubereitung von mizellaren Lösungen mit der einzigen Variante, dass das Arzneimittel wegen seiner Schwerlöslichkeit in Wasser in einer im voraus vorbereiteten mizellaren Lösung gelöst wird; siehe auch: P.L. Luisi und R. Wolf, "Solution behaviour of surfactans" vol. 12, Mittel and Fendler eds., Plenum Press 1982.

Nitrite und Nitrate werden mit Vorteil, auf transdermalem Weg verabreicht. Die erfindungsgemass erhaltichen Gele von umgekehrten Mizellen vorteilhafter als Lösungen, um den Schmerz und die Häufigkeit der anginösen Krisen zu vermindern.

Die aus Vaseline und Lanolinhydrat bestehenden kommerziellen Salben, verwendet als Träger der Nitroglycerine, werden von den Patienten nicht geschätzt, und es scheint ausserdem, dass sie ein konstantes plasmatisches Niveau der Arznei nicht halten können; siehe: S. Sved et al. J. Pharm. Sci., 70,1368 (1981).

Das auf einem geeigneten festen Träger transportierte Gel, Gegenstand dieser Erfindung, ist mit einem Klebeanteil, der den Kontakt mit der Haut sicherstellt, versehen. Dies ist nicht nur die bequemste Gebrauchsform, sondern kann auch die Verteilung der Arznei steuern, um eine konstante Absorption sicherzustellen.

Endopeptidasen werden in der Kosmetik für die stufenweise Eliminierung überflüssiger Haare verwendet. Dabei stellt sich die Schwierigkeit, die Enzyme zum "bulbus pili" kommen zu lassen. Wegen der Fettstoffschicht, die aus der Talgsekretion stammt, kann das "bulbus pili" von einer wässrigen Lösung nicht benetztwerden. Diese Schwierigkeit kann durch die erfindungsgemäss erhältlichen Lösungen von umgekehrten Mizellen in Gelform beseitigt werden, weil durch diese Gele trotz der vorhandenen Fettstoffschicht ein guter Kontakt zwischen dem Enzym und der Basis des Haares gewährleistet wird.

Die nachfolgenden Präparate veranschaulichen die Herstellung von Mikroemulsionen, die als Ausgangsmaterial zur Durchführung des erfindungsgemässen Verfahrens dienen können. Wenn man in die so hergestellten Mikroemulsionen das gelbildende Mittel einverleibt, erhält man erfindungsgemäss die gewünschten Gele.

### Präparate 1 bis 5

1. 0,44 g AOT werden in 10 ml Isopropylpalmitat gelöst, so dass eine 100 mM Lösung des Tensides entsteht.
   Zu dieser Lösung werden mittels einer Mikrospritze 100 Mikroliter einer wässrigen Urogastron-Lösung zugegeben (1 mg/ml in 50 mM Phosphat-Puffer bei einem pH-Wert von 7). Die Zugabe wird langsam durchgeführt, und nach Beendigung der Zugabe wird langsam von Hand gerührt.
   Am Schluss der Operation erhält man eine klare Lösung, deren Gehalt an Urogastron spektrophotometrisch bestimmt werden kann.
2. Eine 6-prozentige Sojalezithin-Lösung wird in gereinigtem Sojaöl bei Raumtemperatur hergestellt. In 10 ml dieser Lösung werden langsam 300 Mikroliter einer wässrigen Lösung von mit Methoxylpolyethylenglykol (PEG, 1 mg/ml) modifiziertem Urogastron eingeführt. Man arbeitet wie oben beschrieben. Die endgültige Lösung enthält 3% Wasser und 0,03 mg/ml Hormon.
3. 6 g Sojalezithin werden in 100 ml gereinigtem Sojaöl gelöst. Zu dieser Lösung werden 7 ml Wasser zugegeben, und es wird von Hand gerührt, bis sich eine klare Mikroemulsion bildet. Es werden dann 20 g Medroxyprogesteronacetat hinzugefügt und es wird bis zu dessen vollständiger Lösung gerührt.
4. 8,8 g AOT werden in 100 ml Isopropylpalmitat gelöst, und zu dieser Lösung werden 6 ml Butan-1-oi als Cotensid zugegeben. Es wird von Hand gerührt, bis eine klare Lösung entsteht, und anschliessend wird bis 45° C erwärmt.
5. AOT wird in Squalan gelöst, so dass man eine 200 mM Lösung an Tensid erhält. Zu 100 ml dieser Lösung gibt man 3 ml einer wässrigen PufferLösung bei pH 8 (Borat 40 mM), die Papain in einer Konzentration von 10 mg/ml enthält, zu.

Die in den Präparaten 1 bis 5 hergestellten umgekehrten Mizellen, beziehungsweise Mikroemulsionen, können erfindungsgemäss durch die Einverleibung des gelbildenden Mittels in die herzustellenden Gele umgewandelt werden.

### Beispiel 1

Die gemäss Präparat 4 hergestellte Lösung von umgekehrten Mizellen wird durch die Zugabe von Gelatine in die Gelform übergeführt.

3 g Gelatine werden in 8 ml Wasser suspendiert. Man erwärmt bis 45° C und nach der Solubilisation wird diese Lösung langsam unter Rühren zu der gemäss Präparation 4 hergestellten Lösung, die ebenfalls eine Temperatur von 45° C aufweist, hinzugefügt.

Nachdem man eine Mikroemulsion erhalten hat, lässt man diese abkühlen und erhält ein Gel. Auf der Oberfläche dieses Gels, welche nicht mit der Haut in Kontakt kommt, wird Nitroglyzerin zugesetzt, welches an Lactose und kolloidalem Silica absorbiert ist, und zwar derart, dass man eine Konzentration an Arzneimittel von 1 mg/cm² hat.

Die erfindungsgemäss hergestellten Gele können ferner solche Präparate zur topischen Anwendung darstellen, die einen Wirkstoff enthalten, der die Bildung von neuem Epithel anregt.

Zur Herstellung der Gele ist es zweckmässig, von Lösungen von umgekehrten Mizellen auszugehen, die in der wässrigen Phase die Peptide, beziehungsweise Proteine, enthalten, insbesondere den epidermische Wachstumsfaktor: EGF epidermal growth factor. Diese Lösungen können, unter Verwendung von biokompatiblen Lösungsmitteln wie Squalen, Miglyol®, Estern von Palmitinsäure (hauptsächlich Isopropylpalmitat), Pflanzenöle, Vaselinöl hergestellt werden. Das Tensid AOT wird für mehrere in diesen Unterlagen angegebene Beispiele verwendet. Bei diesem Verfahren können auch andere Tenside verwendet werden, wie in den Beispielen und in der Ansprüchen gezeigt wird.

In Form von Gelen vorliegende mizellare Lösungen solcher Systeme sind bis jetzt in der Literatur nicht beschrieben worden. Diese sind thermodynamisch stabil und können wertvolle Peptid- und Proteinanteile enthalten. Diese Erfindung beschäftigt sich hauptsächlich mit der Applikation der topischen Anwendung von EGF.

Nach dem erfindungsgemässen Verfahren, kann in solchen Systemen auch dertryptische Kunitzinhibitor (Aprotinin) oder ein Lipoxygenaseinhibitore oder nichtsteroidales Antiphlogistikum oder Natriumhyaluronat solubilisiert wird.

Die Gesamtheit der zwei gelförmigen mizellaren Systeme (eines enthält EGF, das andere den Inhibitor oder das nicht-steroidale Antiphlogistikum oder Natriumhyaluronat) kann als stabile Mischung für therapeutische Zwecke verwendet werden.

Es ist schon bekannt, dass EGF eine Wirkung auf die Augen haben kann; siehe P.N. Patil, Trends in Bioch. Sc., 201 Mai 1984. Ausserdem ist es wohlbekannt, dass die gewöhnlichen Augenlösungen grosse Verluste vom Aktivprinzip bewirken. Diese sind normalerweise wässrige Lösungen und werden leicht durch die Tränensekretion verdünnt und entfernt. Ein öliges Lösungsmittel wäre daher sehr geeignet, in dem es schwieriger ausgewaschen werden kann, aber es ist unmöglich, EGF (oder jedes andere Protein oder Peptid oder hydrophile Verbindung) in öliger Lösungen zu solubilisieren.

Die gelförmigen invertierten Mizellen (oft Mikroemulsionen Wasser-in-Oel benannt) weisen die beschriebenen Nachteile nicht auf. Das EGF wird leicht in die wässrige Mikrophase aufgenommen und kann aus der organi- schen Phase diffundieren. Die vom Tensid verursachte Abnahme der Oberflächenspannung stellt einen guten Kontakt zu der zu pflegenden Oberfläche und eine gleichmässige Verteilung sicher. Mit Stoffen von relativ kleiner Viskosität, wie die obengenannten organischen Lösungsmittel, kann man Tropfen von winzigen Abmessungen bilden.

Dies ist bei der Herstellung von Augentropfen enorm wichtig, weil das Volumen der Augentropfen normalerweise 7 Mikroliter beträgt. Grössere Flüssigkeitsanteile werden sofort aus den Augen ausgestossen. Der grosse Vorteil dieses Systems besteht in der Tatsache, dass der teure biologische Stoff, in unserem Fall EGF, nicht verloren geht, wie im Falle von reinen wässrigen Augentropfen. Ein mit der Therapie vieler Verletzungen verknüpftes Problem ist die Tatsache, dass die proteinasische Aktivität eine Verlangsamung der Genesung bewirkt. Es wurde indirekt bewiesen, dass Proteinaseinhibitoren diese Probleme erleichtern können, weil sie die Proteinasewirkung eliminieren. Wie oben gesagt, können die Inhibitoren leicht in die umgekehrte Mizellen aufgenommen werden. Das Inhibitoren enthaltende System wird in der Therapie als Coadjuvans verwendet.

Man kann auf zwei Wegen die gemischten Systeme vorbereiten: EGF und Inhibitor (z.B. Aprotinin; siehe die Beispiele) werden zusammen zu einerwässrigen Lösung zugegeben, und diese Mischung wird zum mizellaren Kohlenwasserstoffsystem hinzugefügt, wie weiter unten näher beschrieben. Es können auch zwei verschiedene mizellare Lösungen hergestellt werden, die später durchgemischt werden, oder sie können äusserlich nacheinander zugegeben werden, wenn immer möglich in geeigneten und verschiedenen Dosen.

Es ist ausserdem bekannt - siehe: R. Rocheis und W.D. Busse, Graefe'sArch. Clin. Exp. Ophtalmol. 220,74 (1983) -, dass einige Lipoxygenaseinhibitoren die neue Epitelbildung bei Kaninchen, deren Hornepithel ausgekratzt worden ist, beschleunigen. Natriumhyaluronat scheint einen ähnlichen Effekt zu haben. Auch in diesem Fall kann ein gemischtes System die therapeutische Wirkung des EGF günstigen.

Bei der Herstellung der mizellaren Lösungen, die EGF oderAprotinin odercyclooxygenaseninhibitoren oder Natriumhyaluronat enthalten, wird dem klassischen Verfahren zur Herstellung enzymatischer mizellarer Lösungen gefolgt; siehe: R. Wolf und P.L. Luisi, Bioch. Bioph. Res. Comm. 89, 209 (1979). Das Tensid (z.B. AOT) wird im Lösungsmittel gelöst, so dass eine Tensidlösung zwischen 50 mM und 300 mM entsteht. Die Auflösung tritt bei Raumtemperatur und ohne Sonifizierung auf.

Zu dieser Lösung kann man die wässrige EGF-Lösung des Biopolymers zugeben, z.B. 1 ml einer gepufferten Lösung von Phosphat (50 mM pH 7,0) wird zu 100 ml der vorherigen organischen AOT-Lösung zugefügt. Der Anteil von Wasser im System kann bis zu 30%, betragen insbesondere zwischen 0,03% bis 10% (v:v) variieren.

Unter diesen Bedingungen entsteht beim Schütteln von Hand in einigen Minuten und ohne Sonifizierung eine klare und durchsichtige Lösung. Als Lösungsmittel kommen - wie schon erwähnt - niedermolekulare Kohlenwasserstoffe, chlorierte Kohlenwasserstoffe, natürliche und pflanzliche Oele, Squalan, Squalen, Ester von Palmitinsäure und anderen Fettsäuren, Miglyol®, natürliche Essenzen in Frage. Die schlussendliche Konzentration des Biopolymers (in unserem Fall EGF oder ein Proteinaseinhibitor) kann innerhalb der schlussendlichen Lösung in einem breiten Bereich variieren, gemäss derwässrigen Startlösung und dem vorgesehenen Gebrauch. Normalerweise variiert sie zwischen 0,1 - 100 mg/ml, insbesondere 0,1 - 10 mg/ml (Konzentration bezüglich totalem Volumen). Der pH-Wert kann auch breit variiert werden, aber normalerweise werden Lösungen mit einem pH-Wert zwischen 4 und 9 verwendet.

Die auf diese Weise vorbereiteten Lösungen sind für lange Zeit stabil, im besonderen, wenn sie im Kühlschrank (0 - 2° C) aufbewahrt werden. Für eine verlängerte Aufbewahrung werden die Lösungen unter 0° C abgekühlt. Ein Niederschlag wird nur bei höherer Wasserkonzentration (zwischen 5% - 10%) beobachtet, aber das System ist reversibel.

Es ist zu bemerken, dass bei niedriger Wasserkonzentration (unter 3%) das Wasser nicht bei 0° C gefriert.

Die so erhaltenen Lösungen von umgekehrten Mizellen können gemäss einer Ausführungsart des erfindungsgemässen Verfahrens durch Zugabe eines Gelbildners in die erfindungsgemäss hergestellten Gele umgewandelt werden.

In den nachfolgenden Präparaten 6, 7, 8 und 9 wird die Herstellung von weiteren Lösungen von umgekehrten Mizellen erläutert, die als Ausgangsmaterial bei der Durchführung des erfindungsgemässen Verfahrens zur Herstellung der umgekehrten Mizellen in Form von Gelen einsetzbar sind.

### Präparat 6

0,44 g AOT werden in 10 ml Isopropylpalmitat gelöst, so dass eine 100 mM Lösung des Tensides entsteht.

Zu dieser Lösung werden mittels einer Mikrospritze 100 Mikroliter einer wässrigen EGF-Lösung zugegeben (1 mg/ml in 50 mM Phosphat-Puffer bei einem pH-Wert von 7). Die Zugabe wird langsam durchgeführt, und nach Beendigung der Zugabe wird langsam von Hand gerührt.

Am Schluss der Operation erhält man eine klare Lösung, deren Gehalt an EGF spektrophotometrisch bestimmt werden kann.

### Präparat 7

Eine Natriumlaurat (200 mM) Lösung in Squalan mit 10% Hexan-1-oi wird bei Raumtemperaturvorbereitet. In 10 ml dieser Lösung werden langsam 300 Mikroliter einer wässrigen EGF-Lösung (1 mg/1 ml) eingespritzt. Man arbeitet wie oben beschrieben. Die endgültige Lösung enthält 3 % Wasser in 0,03 mg/ml EGF.

### Präparat 8

100 mg BRIT 56@, welches 6% Hexan-1-oi und 1% Wasser enthält, bilden eine Mikroemulsion in Miglyol Shell. Zu 10 ml dieser Lösung werden langsam mit einer Mikrospritze 200 Mikroliter einer EGF-Lösung (1,5 mg/ml) und 200 Mikroliter einer wässrigen Lösung von Aprotinin (Kunitz Inhibitor) zugefügt. Die Mischung wird langsam von Hand gerührt.

Anstatt Aprotinin kann man abwechslungsweise einen Lipoxygenase- oder Natriumlaurat-Inhibitor verwen- den, so dass das gleiche EGF:Coadjuvans molare Verhältnis erhalten bleibt.

### Präparat 9

Tetraethylenglykoldodecylether wird in Squalan gelöst, so dass eine 200 mM Lösung des Tensides entsteht. Zu 100 ml dieser Lösung werden 3 ml einer wässrigen gepufferten Lösung (Borat 40 mM, pH 8,0), die den Kunitzinhibitor (Aprotinin) in einer Konzentration von 10 mg/ml enthält, hinzugefügt.

Die nach den Präparaten 6, 7, 8 und 9 hergestellten Lösungen von umgekehrten Mizellen werden dann erfindungsgemäss in die entsprechenden umgekehrten Mizellen in Gelform umgewandelt, indem man ein gelbildendes Mittel einverleibt, beispielsweise Gelatine. Die Herstellung der entsprechenden Gele kann beispielsweise nach derjenigen Ausführungsart des erfindungsgemässen Verfahrens durchgeführt werden, die im Beispiel 1 beschrieben ist.

Die so erhaltenen Gele werden mit Vorteil in der Pharmakologie, der Biotechnologie und der Kosmetik angewandt.

Bei der Herstellung der umgekehrten Mizellen in Gelform unter Verwendung von Gelatine als gelbildendem Mittel zeigte es sich, dass unter denjenigen Bedingungen, bei denen Gelatine in wässrigen Lösungen geliert, auch in den organischen Lösungsmitteln des Tensides eine Gelierung stattfindet, wobei das gesamte organische Milieu in ein Gel umgewandelt wird.

Es ist bekannt, dass Gelatine in Wasser gelieren kann, und feste Gele bildet, die heutzutage eine grosse technologische Bedeutung, besonders in der Lebensmittelindustrie, haben. Diese Gele sind aber auch in anderen Bereichen der Technologie von Interesse, z.B. in der Photographie oder in all jenen Gebieten, in denen hydrophile Filme oder Schichten wichtig sind. Bekanntlich wird die Gelierung in wässrigen Lösungen so durchgeführt, dass eine Lösung von Gelatine (z.B. 10 mg/ml) bei 40° oder höher hergestellt und dann abgekühlt wird. Unterhalb von etwa 37° findet die Gelierung statt. Dieses Phänomen wurde intensiv untersucht. Auch Polysaccharide können Gele bilden.

Andererseits ist auch bekannt, dass Proteine in apolaren Lösungsmitteln solubilisiert werden können, und zwar durch umgekehrte Mizellen oder Wasser/in Oel Mikroemulsionen. Umgekehrte Mizellen sind spheroidale Aggregate, die von gewissen Tensiden in Kohlenwasserstoffen und anderen organischen apolaren Lösungsmitteln gebildet werden, und zwar so, dass die polaren Köpfe der Tensidmoleküle nach innen gerichtet sind. Somit wird ein polarer Kern in der Mitte des spheroidalen Aggregates gebildet (eben die umgekehrte Mizelle), wobei die apolaren Ketten der Tensidmoleküle nach aussen in Kontakt mit dem apolaren Lösungsmittel stehen. Der polare Kern kann Wasser solubilisieren. Bei höherem Wassergehalt wird dann meist der Begriff "Mikroemulsion" anstatt "umgekehrte (oder invertierte) Mizelle" gebraucht. Im wässrigen Kern (dem sogenannten "water pool") können hydrophile Moleküle, inklusive Proteine, solubilisiert werden. Mit diesen Methoden wurden in den letzten Jahren verschiedene Proteine, z.B. in Isooktan mit (2-Ethyl-hexyl)-Natriumsulfosuc- cinat (AOT) als Tensid solubilisiert, wobei der Wassergehalt im System nur 1-5% betrug. Es steht also ein System zur Verfügung in welchem hydrophile Biopolymere in einem apolaren Lösungsmittel solubilisiert werden können. Eine solche Lösung ist thermodynamisch stabil und kann spektroskopisch analysiert werden. Es können damit normale enzymatische Reaktionen durchgeführt werden.

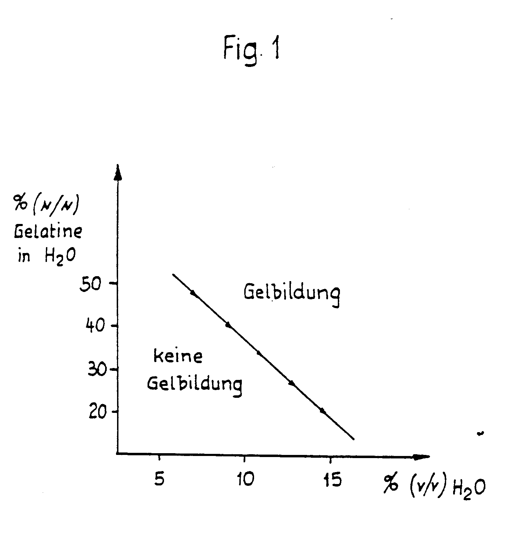

Der Hauptpunkt liegt erfindungsgemäß nun darin, die zwei Verfahren, nämlich die Solubilisierung von in Wasserlöslichen Wirkstoffen, z.B. Proteinen in apolaren Lösungsmitteln und die Gelierung, z.B. der Gelatine, zusammen zu kombinieren. Die Herstellung eines solchen apolaren Gels kann wie folgt durchgeführt werden Gelatine wird in Wasser (oder gepufferte wässrige Lösungen) gegeben und unter Rühren wird eine Lösung von Gelatine in Wasser erhalten. Zur Gelatinelösung wird ein organisches Lösungsmittel (z.B. Isooktan), welches das Tensid enthält, zugegeben. Die Konzentration des Tensids variiert zwischen 0 und 1 M, insbesondere zwischen 5 und 300 mM. Der Volumenanteil des Wassers liegt zwischen 0,5 und 30%. Der Gewichtsanteil von Gelatine in der wässrigen Lösung beträgt 1 - 70%, insbesondere 5 - 50%. Die ganze Operation der Vorbereitung und Mischung der beiden Lösungen erfolgt oberhalb des Gelschmelzpunktes, insbesondere zwischen 20 und 60° C. Beim Abkühlen der resultierenden Mischung entsteht ein Gel. Unter normalen Bedingungen wird der Uebergang bei der gleichen Temperatur beobachtet, bei der die Gelbildung auch in wässriger Lösung erfolgt. Der Vorgang der Gelbildung aus apolaren Lösungen mit umgekehrten Mizellen oder Mikroemulsionen ist reversibel, d.h. das Gel kann beim Erhitzen wieder geschmolzen werden. Unter gewissen Bedingungen, Wassergehalt und Gelatinekonzentration, sind die Gele durchsichtig. Die Gelierung der organischen Phase findet nicht bei jeder beliebigen Kombination von Wassergehalt und Gelatinekonzentration statt. Im allgemeinen gilt, dass wenn der Wassergehalt verkleinert wird, die Gelatinekonzentration dementsprechend erhöht werden muss (siehe Abbildung 1).

Die Bildung des Gels findet auch in Anwesenheit von anderen Molekülen, zusätzlich zur Gelatine, statt. Auf diese Weise können verschiedene Verbindungen in der gelierten Masse homogen verteilt werden. Interessanterweise ist dies der Fall sowohl für hydrophile Moleküle, die im Wasserkern der umgekehrten Mizelle solubilisiert sind, als auch für ganz hydrophobe Moleküle, die im organischen Lösungsmittel ausserhalb der wässrigen Mikrophasen solubilisiert sind. Dies kann auch mit amphi- philen Molekülen stattfinden, d.h. Molekülen, die in der Wand der Mizelle inkorporiert sind.

In den nach dem erfindungsgemässen Verfahren hergestellten, in Form von Gelen vorliegenden umgekehrten Mizellen, ist der Stoff, welcher eine therapeutische oder kosmetische Aktivität aufweist, in der wässrigen Phase, deren Teilchendurchmesser 200 A nicht übersteigt, gelöst. Es ist jedoch möglich, zusätzlich zu diesem Wirkstoff noch weitere Verbindungen, die in dem apolaren organischen Lösungsmittel löslich sind, und auch solche, die sich in der Zwischenphase zwischen Wasser und organischem Lösungsmittel aufhalten, homogen in den entsprechenden Gelen einzubetten.

Nach dem erfindungsgemässen Verfahren kann man hydrophile Substanzen, beispielsweise hydrophile Enzyme oder sogar Zellen in die Gelmasse einbetten. Dies kann von grossem biotechnologischen Interesse sein, weil dieses System eine Methode anbietet, mit welcher Enzyme und Zellen in ein hydrophobes Milieu eingebracht werden können. In die Gelmasse kann man ferner auch Pigmente und Farbstoffe (für die photographische Industrie von besonderer Bedeutung) oder auch Arzneimittel einbetten. Letzteres dürfte für eine kutane Behandlung interessant sein. Schliesslich kann die gelierte Masse auch in der Kosmetik Verwendung finden, wenn ein geeigneter Wirkstoff eingeschlossen wird.

Dieser Vorgang der Gelierung von apolaren Lösungsmitteln ist nicht auf das System Isooktan/AOT beschränkt. Andere aliphatische und aromatische Kohlenwasserstoffe, sowohl synthetischer wie auch natürlicher Herkunft, können als Lösungsmittel dienen. Es können auch Lösungsmittelmischungen verwendet werden. Andere Tenside anionischer, kationischer und auch nicht ionischer Natur (wie diejenigen, die eine Aethylenoxydstruktur besitzen), können ebenfalls benutzt werden. Anstelle von Gelatine können andere Verbindungen verwendet werden, die in wässrigen Lösungen Gele bilden, z.B. Polysaccharide, wie Agarose, Agar-Agar und andere.

Anhand der nachfolgenden Beispiele 2 bis 7, wird eine weitere Ausführungsart zur Durchführung des erfindungsgemässen Verfahrens zur Herstellung der umgekehrten Mizellen in Form von Gelen veranschaulicht.

Während bei der in Beispiel 1 beschriebenen Ausführungsart des erfindungsgemässen Verfahrens zunächst eine Lösung der umgekehrten Mizellen in dem organischen Lösungsmittel hergestellt wird und diese dann mit einer wässrigen Lösung des gelbildenden Mittels gemischt wird, wobei die Vermischung bei einer Temperatur oberhalb der Gelbildungstemperatur vorgenommen wird und anschliessend dann bei Abkühlung die Mischung zu dem Gel erstarrt, wird bei der in den nachfolgenden Beispielen 2 bis 7 beschriebenen Ausführungsart des erfindungsgemässen Verfahrens das Gel hergestellt, indem man zu der den Gelbildner enthaltenden wässrigen Phase direkt das organische Lösungsmittel zugibt, welches das Tensid enthält.

### BEISPIELE

### Beispiel 2

### Herstellung von Gelen aus Kohlenwasserstoffen

220 mg Gelatine (250 Bloom, Fluka) werden unter Rühren in 0,72 ml Wasser gelöst. Zu dieser Gelatinelösung werden 2,5 ml 200 mM AOT/Isooktan-Lösung zugefügt und die Mischung anschliessend mit Isooktan auf total 5 ml verdünnt. Alle diese Operationen werden oberhalb 40° C durchgeführt. Die Lösung wird dann unter Rühren auf Raumtemperatur abgekühlt. Es entsteht ein festes Gel.

### Beispiel 3

### Einschluss eines wasserlöslichen Farbstoffes

Man arbeitet wie in Beispiel 2, ausser dass anstelle von Wasser eine wässrige 1 mM Alizarinrot S Lösung verwendet wird. Es entsteht ein rotes Gel.

### Beispiel 4

### Einschluss eines Isooktan-löslichen Farbstoffes

Man arbeitet wie in Beispiel 2, ausser dass die 2,5 ml 200 mM AOT/Isooktan-Lösung noch 1 mg Sudanrot 7B enthaltet. Es entsteht ein tiefrotes Gel.

### Beispiel 5

### Einschluss eines Wirkstoffes

Man arbeitet wie in Beispiel 2, ausser dass anstelle von Wasser eine wässrige 0,5 mM Vitamin B₁₂ Lösung verwendet wird. Es entsteht ein rosarotes Gel.

### Beispiel 6

### Einschluss eines Aromastoffes

Man arbeitet wie in Beispiel 2, ausser dass anstelle von Wasser eine wässrige 3 mM Lösung von Vanillin verwendet wird. Es entsteht ein farbloses transparentes Gel.

### Beispiel 7

### Einschluss eines Enzyms

Man arbeitet wie in Beispiel 2, ausser dass anstelle von Wasser eine wässrige 10-⁰ M Lipoxygenase Lösung verwendet wird.

## Patentansprüche

1. Verfahren zur Herstellung von Lösungen von umgekehrten Mizellen, deren Teilchendurchmesser der wässrigen Phase 200 Ä nicht übersteigt, die dann auch Mikroemulsionen genannt werden, wenn der Gehalt an Wasser im System relativ gross ist, in Form von Gelen, dadurch gekennzeichnet, dass man in eine Mischung aus wenigstens einem biokompatiblen natürlichen organischen Lösungsmittel und wenigstens einem Tensid, sowie gegebenenfalls einem Cotensid oder einem Coadjuvans, und Wasser, sowie einem in Wasser gelösten Stoff, der eine therapeutische oder kosmetische Aktivität aufweist, ein gelbildendes Mittel einverleibt, vorzugsweise ein gelbildendes Mittel auf Basis eines Polysaccharides oder Gelatine.

2. Verfahren nach Anspruch 1, dadurch gekennzeichnet, dass man als gelbildendes Mittel Gelatine verwendet, beziehungsweise als gelbildendes Mittel auf Basis eines Polysaccharides Agarose oder Agar-Agar verwendet.

3. Verfahren nach Anspruch 1 oder 2, dadurch gekennzeichnet, dass in der Mischung eine oder mehrere zusätzliche Verbindungen vorhanden sind, die bei der Gelierung im Gel festgehalten werden, wobei solche zusätzlichen Verbindungen sowohl hochmolekulare Produkte sein können, wie Proteine, Enzyme, Nukleinsäuren, Zellen, Viren und Mikroorganismen, als auch niedermolekulare Verbindungen wie Pigmente, Farbstoffe, Pharmaka und auch Salze.

4. Verfahren nach einem der Ansprüche 1 bis 3, dadurch gekennzeichnet, dass bei der Herstellung der Gele der Volumenanteil von Wasser im System 0,03 Vol-% bis 30 Vol.-%, bezogen auf das Gesamtvolumen, vorzugsweise 0,03 bis 3 Vol.- %, bezogen auf das Gesamtvolumen, beträgt.

5. Verfahren nach einem der Ansprüche 1-4, dadurch gekennzeichnet, dass das organische Lösungsmittel ausgewählt ist aus Squalan, essentiellen Oelen, Terpenen, Pflanzenölen, Miglyol, Estern von natürlichen Fettsäuren, wie etwa Palmitinsäure, insbesondere Isopropylpalmitat, Stearinsäure, perfluorierten Kohlenwasserstoffen, Vaselinöl, Isooktan.

6. Verfahren nach einem der Ansprüche 1-5, dadurch gekennzeichnet, das das Tensid ausgewählt ist aus Natrium-1,2-bis (2-Ethylhexyloxy- carbonyl)-1-ethansulfonat, abgekürzt als AOT; Benzalconiumchlorid; Cetyltrimethylammoniumbromid, abgekürzt als CTAB; Tensiden auf der Grundlage von anderen quaternären Ammoniumsalzen; Tensiden auf der Grundlage von Ethylenglykol; Tensiden natürlicher Herkunft wie Phospholipide, Lecithine, Phospholecithine, Phosphatidylcholine; synthetischen Tensiden, die als Grundkern Glycerin haben; Tensiden, die die Struktur von Estern von Fettsäuren, wie z.B. Sorbitantristearat oder Polyoxyethylensorbitan Oleat, aufweisen.

7. Verfahren nach einem der Anspruch 1-6, dadurch gekennzeichnet, dass das System Arzneimittel enthält und gebraucht wird, um die Absorption im Darm sowie durch die Haut zu begünstigen.

8. Verfahren nach einem der Ansprüche 1-6, dadurch gekennzeichnet, dass das System Stoffe enthält, die eine kosmetische Wirkung aufweisen und das Eindringen in die Haut begünstigen, z.B. als Coadjuvanten für Enthaarung.

9. Verfahren nach einem der Ansprüche 1-6, dadurch gekennzeichnet, dass das System Verbindungen enthält, die die neue Epithelbildung anregen können oder der Verbrennungsbehandlung dienen.

10. Verfahren nach einem der Ansprüche 1-6, dadurch gekennzeichnet, dass das System den epidemischen Wachstumsfaktor, abgekürzt als EGF, oder andere natürliche oder synthetische Wachstumsfaktoren für therapeutische oder klinische Anwendungen enthält.

11. Verfahren nach einem der Ansprüche 1-6, dadurch gekennzeichnet, dss das System Proteaseninhibitoren oder Lipoxygenase oder nicht steroidale Antiphlogistika oder Natriumhyaluronat enthält.

## Claims

1. Process for the manufacture of solutions of inverted micellae, where the particle diameter in the aqueous phase does not exceed 200 A, which are also called micro-emulsions, when the water content in the system is relatively high, in the form of gels, characterised in that a gel-forming agent, preferably a gel-forming agent based on a polysaccharide or on gelatine is incorporated in a mixture consisting of at least one bio-compatible natural organic solvent and at least one tenside, and, if required, one cotenside or one coadjuvant, and of water as well as a substance dissolved in water, which is therapeutically or cosmetically active.

2. Process according to claim 1, characterised in that the gel-forming agent used is gelatine or in that agarose or agar-agar is used as a polysac- caride-based gel-forming agent.

3. Process according to claim 1 or 2, characterised in that one or more additional compounds are present in the mixture, which compounds are retained in the gel during gelatinisation, whereby such additional compounds can be both products of high molecular weight such as proteins, enzymes, nucleic acids, cells, viruses and micro organisms, and compounds of low molecular weight such as pigments, dyes, pharmaceuticals and salts.

4. Process according to one of claims 1 to 3, characterised in that with the manufacture of the gels the volume percent of water in the system is 0.03% by vol. to 30% by vol. relative to the total volume.

5. Process according to one of claims 1 - 4, characterised in that the organic solvent is selected from squalane, essential oils, terpenes, vegetable oils, miglyol, esters from natural aliphatic acids such as palmitic acid, especially isopropyl palmitate, stearic acid, perfluorised hydrocarbons, vaseline oil, isooctane.

6. Process according to one of claims 1 to 5, characterised in that the tenside is selected from sodium 1,2-bis(2-ethyl hexyl oxycarbonyl) -1-ethane sulphonate, abbreviated to AOT; benzalconium chloride; cetyl trimethyl ammonium bromide, abbreviated to CTAB; tensides on the basis of other quaternary ammonium salts; tensides on the basis of ethylene glycol; tensides of natural origin such as phosphatides, lecithines, phos- pholecithines, phosphatidylcholines; synthetic tensides which are based on glycerine; tensides which have the structure of aliphatic acid esters such as sorbitan tristearate or polyoxyethylene sorbitan oleate.

7. Process according to one of claims 1 to 6, characterised in that the system contains drugs and is used to encourage the absorption in the gut as well as through the skin.

8. Process according to one of claims 1 to 6, characterised in that the system contains substances which are of cosmetic effect and encourage penetration into the skin, e.g. as coadjuvants for the removal of hair.

9. Process according to one of claims 1 - 6, characterised in that the system contains compounds, which can stimulate the formation of new epithelium or can be used for the treatment of burns.

10. Process according to one of claim 1 - 6, characterised in that the system contains the epimedic growth factor abbreviated to EGF or other natural or synthetic growth factors for therapeutic orclin- ical applications.

11. Process according to one of claims 1 to 6, characterised in that the system contains proteinase inhibitors or lipoxygenases or non-steroidal anti- phlogistica or sodium hyaluronate.

## Revendications

1. Procédé de préparation de solutions de micelles inversées, dont le diamètre des particules de la phase aqueuse ne dépasse pas 200 Aetqu! sont également appelées micro-émulsions lorsque la teneur en eau du système est relativement forte, sous forme de gels, caractérisé en ce qu'on incorpore dans un mélange d'au moins un solvant organique naturel biocompatible et d'au moins un tenside avec, le cas échéant, un co-tenside ou un co-adjuvant, et de l'eau ainsi qu'une substance dissoute dans de l'eau, qui présente une activité thérapeutique ou cosmétique, un milieu gélifiant, de préférence un milieu gélifiant à base d'un polysaccharide ou de la gélatine.

2. Procédé selon la revendication 1, caractérisé en ce qu'on utilise comme milieu gélifiant de la gélatine ou on utilise comme milieu gélifiant à base de polysaccharide de l'agarose ou de l'agar-agar.

3. Procédé selon la revendication 1 ou 2, caractérisé en ce que sont présents dans le mélange un ou plusieurs composés supplémentaires qui sont retenus dans le gel lors de la gélification, de tels composés supplémentaires pouvant être aussi bien des produits de poids moléculaire élevé, tels que des protéines, des enzymes, des acides nucléiques, des cellules, des virus et des micro-organismes, que des composés de bas poids moléculaires, tels que des pigments, des colorants, des produits pharmaceutiques et même des sels.

4. Procédé selon l'une quelconque des revendications 1 à 3, caractérisé en ce que, lors de la préparation des gels, la part volumique en eau dans le système s'élève à 0,03 à 30% en volume, par rapport au volume total, de préférence à 0,03 à 3% en volume, par rapport au volume total.

5. Procédé selon l'une quelconque des revendic- tions 1 à 4, caractérisé en ce que le solvant organique est choisi parmi le squalane, les huiles essentielles, les terpènes, les huiles végétales, le Miglyol, les esters d'acides gras naturels, comme par exemple de l'acide palmitique, en particulier le palmitate d'isopropyle, l'acide stéarique, les hydrocarbures perfluorés, l'huile de vaseline ou l'iso-octane.

6. Procédé selon l'une quelconque des revendications 1 à 5, caractérisé en ce que le tenside est choisi parmi le 1,2-bis-(2-éthylhexyloxycarbo- nyl)-1-éthanesulfonate de sodium désigné par l'abréviation AOT, le chlorure de benzalconium, le bromure de cétyltriméthylammonium désigné pr l'abréviation CTAB; les tensides à base d'autres sels d'ammonium quaternaire; les tensides à base d'éthylèneglycol; les tensides d'origine naturelle, tels que les phospholipides, les lécithines, les phospholécithines, les phosphatidylcholines; les tensides synthétiques qui comportent de la glycérine comme élément de base; ou les tensides qui présentent la structure d'esters d'acides gras, comme par exemple le tristéarate de sorbitanne ou un oléate de polyoxyéthylène- sorbitanne.

7. Procédé selon l'une quelconque des revendications 1 à 6, caractérisé en ce que le système contient des substances médicamenteuses et est utilisé pourfavoriser leur absorption dans l'intestin, ainsi qu'à travers la peau.

8. Procédé selon l'une quelconque des revendications 1 à 6, caractérisé en ce que le système contient des substances qui présentent une activité cosmétique et qui favorisent la pénétration dans la peau, par exemple des enzymes servant de co-adjuvants pour l'épilation.

9. Procédé selon l'une quelconque des revendications 1 à 6, caractérisé en ce que le système contient des composés qui peuvent exciter la régénération de l'épithélium ou qui servent au traitement des brûlures.

10. Procédé selon l'une quelconque des revendications 1 à 6, caractérisé en ce que le système contient le facteur de croissance épidermique, désigné par l'abréviation EGF, ou d'autres facteurs de croissance naturels ou synthétiques pour des applications thérapeutiques ou cliniques.

11. Procédé selon l'une quelconque des revendications 1 à 6, caractérisé en ce que le système contient des inhibiteurs de protéinases, des li- poxygénases, des antiphlogistiques non stéroïdes ou de l'hyaluronate de sodium.
